# EUROPÄISCHE PATENTSCHRIFT

(11) **EP 1 110 579 B1**
(45) Veröffentlichungstag und Bekanntmachung des Hinweises auf die Patenterteilung: **04.05.2005**
(21) Anmeldenummer: 00250432.2
(22) Anmeldetag: 12.12.2000
(51) Int. Cl.: A61N 1/05, A61B 5/042

(54) **Multipolare Elektrodenanordnung**
Multipolar electrode device
Dispositif à électrode multipolaire

(30) Priorität: 23.12.1999 DE 19963603
(43) Veröffentlichungstag der Anmeldung: 27.06.2001
(73) Patentinhaber: BIOTRONIK Mess- und Therapiegeräte GmbH & Co Ingenieurbüro Berlin, 12359 Berlin (DE)
(72) Erfinder: Steglich, Carsten, 10407 Berlin (DE)
(74) Vertreter: Eisenführ, Speiser & Partner

(56) Entgegenhaltungen:
- US-A- 5 394 880
- US-A- 5 433 742
- US-A- 5 921 923
- US-A- 5 941 834

## Beschreibung

Die Erfindung betrifft eine multipolare Elektrodenanordnung mit mehreren Elektroden mit elektrischer Zuleitung.

Ein besonderes Einsatzgebiet multipolarer Elektrodenanordnungen ist die Abgabe elektrischer Signale an Körpergewebe, insbesondere Herzgewebe, sowie die Aufnahme elektrischer Signale des Herzens. Die Kontraktion und Entspannung der Herzmuskeln wird durch elektrische Signale gesteuert, die in einer Erregungsfront das Herzgewebe durchlaufen. Für die Diagnose und Therapie von Herzerkrankungen sind Erkenntnisse über die Signalausbreitung im Herzen von großer Bedeutung. Aus dem US-Patent 5,921,923 ist eine multipolare Elektrodenanordnung bekannt, bei der die Elektroden so angeordnet sind, daß nicht nur das Auftreten von Ereignissen, sondern zusätzlich auch Richtung und Geschwindigkeit der Reizleitung erfaßt werden können.

Dazu sind die Pole auf dem Katheter räumlich so zueinander angeordnet, daß jeweils Paare davon gleichsam ein Koordinatensystem aufspannen. Andere Elektrodenanordnungen gehen aus den US-Patentschriften 5,385,146 und 5,476,503 hervor.

Aufgabe der vorliegenden Erfindung ist es, eine Elektrodenanordnung mit alternativem Aufbau anzugeben.

Gelöst wird diese Aufgabe durch eine Elektrodenanordnung der eingangs genannten Art, bei der die Elektroden mit einem Elektrodenträger verbunden sind, der zusammen mit den Elektroden in das Lumen eines Katheters einführbar gestaltet ist.

Eine derartige Elektrodenanordnung erlaubt eine vereinfachte Herstellung beliebiger Polkonfigurationen insbesondere auf einem Single lead-Elektrophysiologiekatheter. Die Konfiguration der Elektroden ist dabei durch den Elektrodenträger vorgegeben. Der Elektrodenträger mit den daran befestigten Elektroden kann vormontiert und anschließend samt elektrischer Zuleitungen in einen Katheterschlauch eingeschoben werden.

Die Elektrodenanordnung umfaßt einen Katheter mit einem Lumen, das zur Aufnahme des Elektrodenträgers gestaltet ist, wobei der Katheter von dem Lumen ausgehende Öffnungen aufweist, die derart angeordnet und gestaltet sind, daß die mit dem Elektrodenträger verbundenen Elektroden elektrische Signale außerhalb des Katheters aufnehmen können. Die Öffnungen können sich in einer bevorzugten Variante in der Umfangsfläche des Katheters und insbesondere eines Katheterschlauches befinden, und zwar derart, daß für jede einzelne Elektrode eine entsprechende Öffnung vorgesehen ist. Alternativ kann der Elektrodenträger aber auch so ausgeführt sein, daß er als Ganzes aus einer zentralen Öffnung am distalen Ende des Katheters oder Katheterschlauches herausragt.

Bevorzugt ist eine Elektrodenanordnung, bei der der Elektrodenträger elastisch verformbar ist, und zwar vorzugsweise im wesentlichen in einer ersten Ebene, während er in einer zu der ersten Ebene senkrechten zweiten Ebene wesentlich steifer ist. Dazu umfaßt der Elektrodenträger vorzugsweise ein Blattfederelement mit vorzugsweise flachem Querschnittsprofil.

Das Blattfederelement ist vorzugsweise elektrisch isolierend ausgeführt und besitzt vorzugsweise ein flaches Querschnittprofil. In einer bevorzugten Ausführungsform besteht der Elektrodenträger und insbesondere das Blattfederelement zumindest teilweise aus einem Polymer. Der Elektrodenträger kann dann in besonders günstiger Weise als Spritzteil (injection molding) ausgeführt werden.

Die Vorzüge einer Elektrodenanordnung mit einem solchen Elektrodenträger kommen insbesondere dann zum Tragen, wenn der Elektrodenträger an seinem distalen Ende mit einem Steuermittel verbunden ist, das entlang des Elektrodenträgers relativ zu diesem längsverschieblich geführt ist, so daß durch Längsverschieben des Steuermittels relativ zum Elektrodenträger eine Auslenkung des Elektrodenträgers an dessen distalem Ende bewirkt werden kann. Auf diese Weise läßt sich ein Elektrophysiologiekatheter erzielen, der in Hohlräumen wie beispielsweise dem Atrium oder Ventrikel eines Herzens gezielt ausgelenkt und in eine definierte Richtung geführt werden kann, um Signale an definierten Orten des Herzens aufnehmen zu können. Dazu kann die Katheterspitze mit Markierungsmitteln versehen sein, die es erlauben, die Katheterspitze von außerhalb des Körpers mit Hilfe bildgebender Verfahren zu orten.

Bevorzugt ist eine Elektrodenanordnung, bei der die Elektroden in Längs- und Umfangsrichtung des Kathethers derart zueinander versetzt angeordnet sind, daß sich wenigstens eine Elektrodenmatrix ergibt, die es erlaubt, Richtung und Geschwindigkeit eines Signals aus dem Zeitversatz zu bestimmen, mit dem das Signal verschiedene der Elektroden erreicht. Für die Bestimmung der Geschwindigkeit der Reizleitung ist hierbei ein ausreichender Abstand nicht nur der Flächenmittelpunkte der einzelnen Elektroden, sondern zwischen den Elektrodenflächen, vorteilhaft.

Eine Elektrodenmatrix umfaßt bei der zuvor genannten Elektrodenanordnung vorzugsweise wenigstens drei Elektroden, wobei die Flächenmittelpunkte der Elektroden einer Elektrodenmatrix vorzugsweise auf den Eckpunkten gedachter Drei- oder Vierecke angeordnet sind.

Alternativ zu einer Ausführung der Elektroden aus Metall, die den Vorteil der hohen Leitfähigkeit von Metall bietet, wird eine Elektrodenanordnung besonders bevorzugt, bei der die Elektroden leitfähigen Kunststoff umfassen.

Eine derartige Elektrodenanordnung erlaubt es in einer vorteilhaften Ausführungsform, die Elektroden in Vertiefungen eines nicht leitenden Basismaterials, insbesondere dem isolierenden Blattfederelement, anzuordnen. Diese Vertiefungen können beispielsweise sehr präzise mit einem Laserstrahl hergestellt werden, so daß selbst kleine Mikrostrukturen erzeugt werden können. Das nicht leitende Basismaterial kann Bestandteil des Elektrodenträgers sein.

Grundsätzlich sind Elektroden aus leitendem Kunststoff aus den US-Patentschriften 5,433,742 und 5,029,585 bekannt.

Die Erfindung soll nun anhand von Ausführungsbeispielen mit Hilfe der Figuren näher erläutert werden. Von den Figuren zeigen:
- Figur 1: Einen Elektrodenträger samt Elektroden und Zuleitungen in der Seitenansicht;
- Figur 2: Den Elektrodenträger aus Figur 1 in der Aufsicht;
- Figur 3: Den Elektrodenträger aus Figur 1 und 2 eingesetzt in einen Ein-Lumen-Schlauch eines Elektrophysiologiekatheters;
- Figur 4: Eine Ansicht ähnlich Figur 2 eines Elektrodenträgers mit alternativer Elektrodenanordnung;
- Figur 5: Einen Elektrodenträger mit einer zweiten alternativen Elektrodenanordnung.

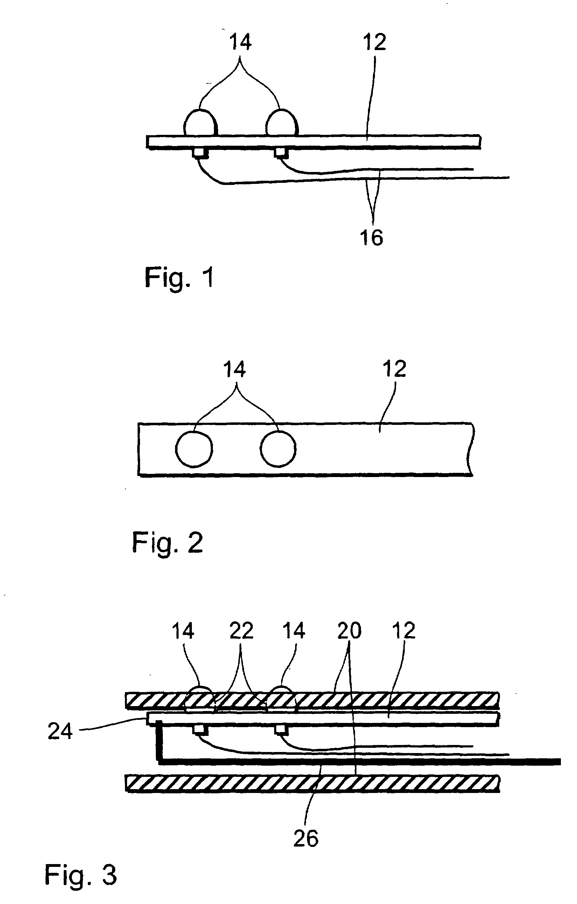

Figur 1 zeigt einen Elektrodenträger 10, dessen wesentlicher Bestandteil ein isolierendes Blattfederelement 12 ist. An dem isolierenden Blattfederelement 12 sind zwei Elektroden 14 befestigt, indem die Elektroden 14 mit dem Blattfederelement 12 verklebt oder vernietet sind. Jeder Elektrode 14 ist jeweils eine elektrische Zuleitung 16 zugeordnet. Die Elektroden bestehen aus Platin, Iridium oder aus elektrisch leitfähigem Kunststoff.

Figur 2 zeigt den Elektrodenträger aus Figur 1 in der Aufsicht und damit die Anordnung der Elektroden 14 auf dem isolierenden Blattfederelement 12. Die Elektroden 14 sind mit einem Abstand voneinander auf der Längsachse des isolierenden Blattfederelementes 12 angeordnet. Durch den Abstand der Elektroden 14 voneinander erreicht ein Signal mit einer Ausbreitungskomponente in Längsrichtung des Blattfederelementes die eine Elektrode 14 vor der anderen. Auf diese Weise kann die Geschwindigkeitskomponente der Signalausbreitung in Längsrichtung des Blattfederelementes aus der Verzögerung bestimmt werden, mit der das Signal von den beiden Elektroden 14 erfaßt wird.

In Figur 3 ist der Elektrodenträger 10 aus den Figuren 1 und 2 in einen Ein-Lumen-Schlauch 20 eines Elektrophysiologiekatheters eingesetzt. Der Schlauch besitzt zwei Öffnungen 22, die zur Aufnahme der Elektroden 14 vorgesehen sind, so daß diese aus dem Lumen des Schlauches nach außen ragen, um elektrische Signale außerhalb des Schlauches aufnehmen zu können.

Außerdem ist am distalen Ende 24 des Blattfederelementes eine Steuermechanik 26 befestigt, die beispielsweise von einem Draht gebildet sein kann, der mit Ausnahme des Ortes der Befestigung am distalen Ende 24 des Blattfederelementes 12 in Längsrichtung des Blattfederelementes 12 relativ zu diesem verschiebbar ist. Dadurch kann der Elektrophysiologiekatheter im Bereich des Blattfederelementes 12 nach Art an sich bekannter steuerbarer Führungsdrähte gezielt seitlich ausgelenkt werden.

Das in den Figuren 1 bis 3 verwirkliche Elektrodenträgerkonzept ermöglicht eine einfache Anordnung von multipolaren Elektroden. Ein großer Vorteil ist eine einfache Handhabung in der Fertigung. Elektrodenträger samt der Elektroden 14 können als eine Baugruppe komplett vormontiert werden und anschließend in den Ein-LumenSchlauch 20 eingeführt werden. Das isolierende Blattfederelement 12 hat eine rechteckige Querschnittsform, die in eine durch die Längsachse des Blattfederelementes 12 verlaufende Ebene für eine große Seitenstabilität sorgt, während sie ein elastisches Auslenken des Blattfederelementes 12 in einer zu der ersten Ebene senkrechten Ebene erlaubt.

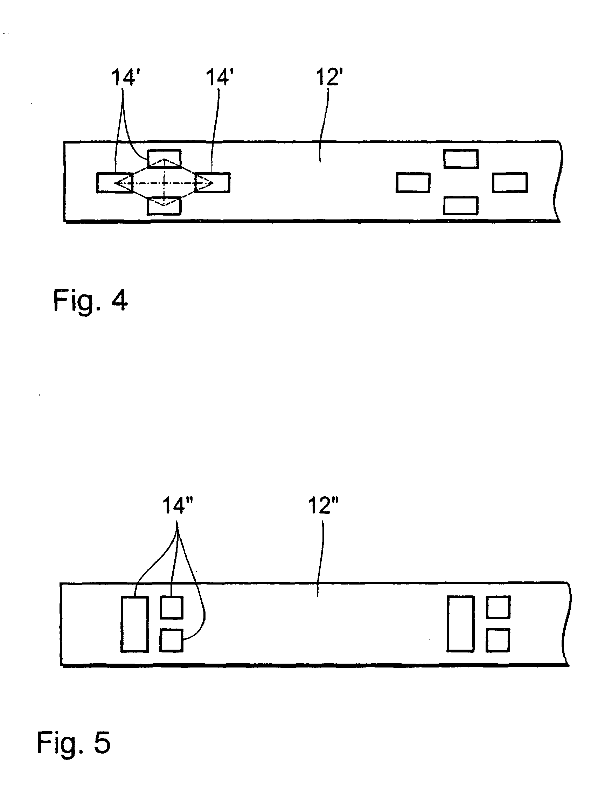

Figuren 4 und 5 zeigen jeweils alternative Elektrodenanordnungen. In Figur 4 sind insgesamt acht Elektroden 14' auf dem Blattfederelement 12' angeordnet. Jeweils vier Elektroden 14' sind zu einer Elektrodenmatrix 30 zusammengefaßt. Die Elektroden 14' einer Elektrodenmatrix sind mit einem Abstand voneinander derart angeordnet, daß ihre Flächenmittelpunkte auf den Eckpunkten eines gedachten Vierecks liegen. Dieses gedachte Viereck ist bezüglich derjenigen zwei Achsen spiegelsymmetrisch, die die jeweils nicht benachbarten Eckpunkte des Vierecks verbinden. Diese beiden Achsen stehen im übrigen senkrecht aufeinander und eine dieser Achsen verläuft in Richtung der Längsachse des Federelementes 12. Das gedachte Viereck ist in Figur 4 gestrichelt eingezeichnet und die beiden die Eckpunkte verbindenden Achsen strichpunktiert.

In Figur 5 werden die beiden Elektrodenmatrizen 30' von jeweils drei Elektroden 14" gebildet. Die drei Elektroden 14" einer Elektrodenmatrix 30' haben nicht sämtlich die gleiche Fläche wie die Elektroden 14' aus Figur 4. Vielmehr besitzt eine der Elektroden 14" eine doppelt so große Fläche, wie die beiden anderen Elektroden 14" der gleichen Elektrodenmatrix 30'. Die beiden kleineren Elektroden 14" sind zu beiden Seiten der gestrichelt gezeichneten Längsachse des Blattfederelementes 12" nebeneinander angeordnet. Die dritte, größere Elektrode 14" ist den beiden kleineren Elektroden 14" in Längsrichtung des Blattfederelementes 12" benachbart angeordnet.

Die Elektrodenanordnungen in den Figuren 4 und 5 weisen eine Reihe gemeinsamer Merkmale auf: Die einzelnen Elektroden 14', 14" einer Elektrodenmatrix 30, 30' haben untereinander einen Abstand, der ausreichend bemessen ist, so daß ein fortschreitendes Signal eine oder zwei der Elektroden der Elektrodenmatrix eher erreicht als die übrigen Elektroden. Aus dem Zeitversatz, mit der ein Signal die einzelnen Elektroden einer Elektrodenmatrix 30 erreicht, sowie aus der Anordnung der Elektroden innerhalb der Elektrodenmatrix 30, 30' kann sowohl die Ausbreitungsreichtung als auch die Ausbreitungsgeschwindigkeit des Signals bestimmt werden.

Weiterhin sind jeweils zwei Elektrodenmatrizen 30, 30' vorgesehen, die voneinander in Längsrichtung des Blattfederelementes 12', 12" einen wesentlich größeren Abstand haben, als die Elektroden 14', 14" einer Elektrodenmatrix 30, 30' untereinander haben.

Die Elektroden 14' und 14" der in den Figuren 4 und 5 abgebildeten Elektrodenanordnungen werden von Vertiefungen in dem isolierenden Blattfederelement 12', 12" gebildet, die mit leitfähigem Kunststoff ausgefüllt sind. Zum Herstellen solcher Elektroden werden zunächst beispielsweise mit Hilfe eines Lasers die Vertiefungen in dem isolierenden Blattfederelement 12 oder 12' erzeugt. Dies kann mit sehr großer Genauigkeit geschehen. Anschließend werden diese Vertiefungen mit leitfähigem Kunststoff ausgefüllt. Die auf diese Art erzeugten Elektroden weisen eine große Genauigkeit auf. Gleichzeitig ist die Herstellungsweise besonders einfach. Diese Art der Elektrodenausführung kann daher auch in anderem Zusammenhang vorteilhaft verwendet werden und ist nicht auf die Anwendungen im Zusammenhang mit dem beschriebenen Elektrodenträger beschränkt.

## Patentansprüche

1. Multipolare Elektrodenanordnung mit einem Katheter (20) und mehreren Elektroden (14, 14', 14") mit elektrischer Zuleitung (16), **dadurch gekennzeichnet, dass** die Elektroden (14, 14', 14") mit einem Elektrodenträger (10) verbunden sind, der zusammen mit den Elektroden (14, 14', 14") in das Lumen des Katheters (20) einführbar gestaltet ist, dass der Katheter (20) in der Umfangsfläche von dem Lumen ausgehende Öffnungen (22) aufweist, die derart angeordnet und gestaltet sind, dass die mit dem Elektrodenträger (10) verbundenen Elektroden (14) elektrische Signale außerhalb des Katheters (20) aufnehmen oder abgeben können, und dass der Elektrodenträger (10) mit den daran befestigten Elektroden (14) vormontiert und anschließend samt elektrischer Zuleitungen (16) in den Katheterschlauch eingeschoben werden kann.

2. Elektrodenanordnung nach Anspruch 1, **dadurch gekennzeichnet, dass** der Elektrodenträger (10) elastisch verformbar ist.

3. Elektrodenanordnung nach Anspruch 2, **dadurch gekennzeichnet, dass** der Elektrodenträger (10) im wesentlichen in einer ersten Ebene elastisch verformbar ist, während er in der zu der ersten Ebene senkrechten zweiten Ebene wesentlich steifer ist.

4. Elektrodenanordnung nach einem der Ansprüche 1 bis 3, **dadurch gekennzeichnet, dass** der Elektrodenträger (10) ein isolierendes Blattfederelement (12) umfasst.

5. Elektrodenanordnung nach Anspruch 4, **dadurch gekennzeichnet, dass** das Blattfederelement (12) ein flaches Querschnittsprofil besitzt.

6. Elektrodenanordnung nach einem der Ansprüche 1 bis 5, **dadurch gekennzeichnet, dass** der Elektrodenträger und insbesondere das Blattfederelement zumindest teilweise aus einem Polymerwerkstoff bestehen.

7. Elektrodenanordnung nach einem der Ansprüche 1 bis 6, **gekennzeichnet durch** ein Steuermittel (26), das entlang dem Elektrodenträger (10) relativ zu diesem längsverschieblich geführt und am distalen Ende (24) mit dem Elektrodenträger (10) derart verbunden ist, dass **durch** Längsverschieben des Steuermittels (26) relativ zum Elektrodenträger (10) eine Auslenkung des Elektrodenträgers (10) an dessen distalem Ende (24) bewirkbar ist.

8. Elektrodenanordnung nach einem der Ansprüche 1 bis 7, **dadurch gekennzeichnet, dass** die Elektroden in Längs- und Umfangsrichtung des Katheters (20) derart zueinander versetzt angeordnet sind, dass sich wenigstens eine Elektrodenmatrix (30, 30') ergibt, die es erlaubt, Richtung und Geschwindigkeit eines aufzunehmenden Signals aus dem Zeitversatz zu bestimmen, mit dem das Signal verschiedene der Elektroden erreicht oder Richtung und Geschwindigkeit eines abzugebenden Signals zu definieren.

9. Elektrodenanordnung nach Anspruch 8, **dadurch gekennzeichnet, dass** eine Elektrodenmatrix (30, 30') wenigstens drei Elektroden (14', 14") umfasst.

10. Elektrodenanordnung nach Anspruch 8 oder 9, **dadurch gekennzeichnet, dass** die Flächenmittelpunkte der Elektroden (14', 14") einer Elektrodenmatrix (30, 30') auf den Eckpunkten gedachter Drei- oder Vierecke angeordnet sind.

11. Elektrodenanordnung nach einem der Ansprüche 1 bis 10, **dadurch gekennzeichnet, dass** die Elektroden (14', 14") leitfähigen Kunststoff umfassen.

12. Elektrodenanordnung nach Anspruch 11, **dadurch gekennzeichnet, dass** Elektroden (14', 14") in Vertiefungen eines nichtleitenden Basismaterials des Elektrodenträgers (10) angeordnet sind.

## Claims

1. Multipolar electrode arrangement comprising a catheter (20) and a plurality of electrodes (14, 14', 14") with an electrical feed line (16), **characterised in that** the electrodes (14, 14', 14") are connected to an electrode carrier (10), which is configured such that it may be inserted, together with the electrodes (14, 14', 14"), into the lumen of the catheter (20), and **in that** the catheter (20) comprises openings (22) that extend in the peripheral surface from the lumen and are arranged and configured such that the electrodes (14) connected to the electrode carrier (10) can pick up or issue electrical signals outside the catheter (20), and **in that** the electrode carrier (10) with the electrodes (14) attached thereto may be pre-assembled and inserted, together with the electrical feed line (16) into the catheter tube.

2. Electrode arrangement according to claim 1, **characterised in that** the electrode carrier (10) is elastically deformable.

3. Electrode arrangement according to claim 2, **characterised in that** the electrode carrier (10) is elastically deformable substantially in a first plane, while it is substantially more rigid in the second plane, which is perpendicular to the first plane.

4. Electrode arrangement according to any one of claims 1 to 3, **characterised in that** the electrode carrier (10) comprises an insulating leaf spring element (12).

5. Electrode arrangement according to claim 4, **characterised in that** the leaf spring element (12) has a flat cross-sectional profile.

6. Electrode arrangement according to any one of claims 1 to 5, **characterised in that** the electrode carrier and in particular the leaf spring element are made, at least in part, of a polymer material.

7. Electrode arrangement according to any one of claims 1 to 6, **characterised by** a control means (26), which is guided in a longitudinally displaceable manner along the electrode carrier (10) relative thereto and at the distal end (24) is connected to the electrode carrier (10) such that deflection of the electrode carrier (10) at the distal end (24) thereof may be effected by means of longitudinal displacement of the control means (26) relative to the electrode carrier (10).

8. Electrode arrangement according to any one of claims 1 to 7, **characterised in that** the electrodes are arranged in a mutually offset manner in the longitudinal and peripheral directions of the catheter (20), so as to produce at least one electrode matrix (30, 30'), which allows the direction and speed of a signal to be picked up to be determined from the time offset with which the signal reaches various of the electrodes, or the direction and speed of a signal to be issued to be defined.

9. Electrode arrangement according to claim 8, **characterised in that** an electrode matrix (30, 30') comprises at least three electrodes (14', 14").

10. Electrode arrangement according to either claim 8 or claim 9, **characterised in that** the centre points of the surfaces of the electrodes (14', 14") of an electrode matrix (30, 30') are arranged at the comers of notional triangles or rectangles.

11. Electrode arrangement according to any one of claims 1 to 10, **characterised in that** the electrodes (14', 14") comprise conductive plastics material.

12. Electrode arrangement according to claim 11, **characterised in that** electrodes (14', 14") are arranged in depressions in a non-conductive base material of the electrode carrier (10).

## Revendications

1. Dispositif multipolaire à électrodes comportant un cathéter (20) et plusieurs électrodes (14, 14', 14") pourvues d'un conducteur d'alimentation électrique (16), **caractérisé en ce que** les électrodes (14, 14', 14") sont reliées à un porte-électrodes (10), qui est agencé de manière à pouvoir être introduit, conjointement avec les électrodes (14, 14', 14"), dans la lumière du cathéter (20), que le cathéter (20) possède dans la surface circonférentielle, des ouvertures (22) qui s'étendent à partir de la lumière et sont disposées et conformées de telle sorte que les électrodes (14) reliées au porte-électrodes (10) peuvent recevoir ou délivrer des signaux électriques à l'extérieur du cathéter (20) et que le porte-électrodes (10) peut être préassemblé avec les électrodes (14) fixées sur ce porte-électrodes et être ensuite inséré, conjointement avec les canalisations d'alimentation électrique (16) dans le tuyau du cathéter.

2. Dispositif à électrodes selon la revendication 1, **caractérisé en ce que** le porte-électrodes (10) est déformable élastiquement.

3. Dispositif à électrodes selon la revendication 2, **caractérisé en ce que** le porte-électrodes (10) peut être déformé élastiquement sensiblement dans un premier plan alors qu'il est nettement plus rigide dans le second plan perpendiculaire au premier plan.

4. Dispositif à électrodes selon l'une des revendications 1 à 3, **caractérisé en ce que** le porte-électrodes (10) comprend un élément de ressort à lame isolant (12).

5. Dispositif à électrodes selon la revendication 4, **caractérisé en ce que** l'élément de ressort à lame (12) possède un profil plat en coupe transversale.

6. Dispositif à électrodes selon l'une des revendications 1 à 5, **caractérisé en ce que** le porte-électrodes et notamment l'élément de ressort à lame sont constitués au moins en partie par un matériau polymère.

7. Dispositif à électrodes selon l'une des revendications 1 à 6, **caractérisé par** un moyen de commande (26), qui est guidé de manière à être déplaçable longitudinalement le long du porte-électrodes (10) par rapport à ce dernier et est relié, au niveau de l'extrémité distale (24) au porte-électrodes (10) de telle sorte que sous l'effet du déplacement longitudinal du moyen de commande (26) par rapport au porte-électrodes (10), une déviation du porte-électrodes (10) peut être réalisé au niveau de son extrémité distale (24).

8. Dispositif à électrodes selon l'une des revendications 1 à 7, **caractérisé en ce que** les électrodes sont disposées en étant décalées réciproquement dans la direction longitudinale et dans la direction périphérique du cathéter (20) de telle sorte qu'on obtient au moins une matrice d'électrodes (30, 30') qui permet de déterminer la direction et la vitesse d'un signal devant être reçu, à partir du décalage temporel, avec lequel le signal atteint différentes électrodes, ou de définir une direction et une vitesse d'un signal devant être délivré.

9. Dispositif à électrodes selon la revendication 8, **caractérisé en ce qu'**une matrice d'électrodes (30, 30') comprend au moins trois électrodes (14, 14").

10. Dispositif à électrodes selon la revendication 8 ou 9, **caractérisé en ce que** les centres des faces des électrodes (14', 14") d'une matrice d'électrodes (30, 30') sont disposés au niveau de sommets de triangles ou de carrés imaginaires.

11. Dispositif à électrodes selon l'une des revendications 1 à 10, **caractérisé en ce que** les électrodes (14', 14") comprennent une matière plastique conductrice (12).

12. Dispositif à électrodes selon la revendication 11, **caractérisé en ce que** les électrodes (14', 14") sont disposées dans des renfoncements d'un matériau de base non conducteur du porte-électrodes (10).
